# EUROPÄISCHE PATENTSCHRIFT

(11) **EP 3 634 342 B1**
(45) Veröffentlichungstag und Bekanntmachung des Hinweises auf die Patenterteilung: **17.09.2025**
(21) Anmeldenummer: 18740498.3
(22) Anmeldetag: 08.06.2018
(51) Int. Cl.: A61F 13/00, A61F 13/02, A61F 13/0246, A61F 13/10, A61K 8/02, A61K 8/65, A61K 8/73, A61K 8/891, A61K 9/70, A61Q 19/00, A61Q 19/08

(54) **SILIKONPAD MIT WIRKSTOFFEN ZUR HAUTPFLEGE UND GEGEN HAUTALTERUNG**
SILICONE PAD HAVING ACTIVE INGREDIENTS FOR SKIN CARE AND AGAINST SKIN AGING
COUSSINET EN SILICONE COMPRENANT DES PRINCIPES ACTIFS POUR LES SOINS DE LA PEAU ET CONTRE LE VIEILLISSEMENT DE LA PEAU

(30) Priorität: 08.06.2017 DE 102017209702; 07.07.2017 DE 102017211702; 23.01.2018 DE 102018101500
(43) Veröffentlichungstag der Anmeldung: 15.04.2020
(73) Patentinhaber: APRICOT GMBH, 85598 Baldham (DE)
(72) Erfinder: JÄGER, Verena, 85661 Forstinning (DE)
(74) Vertreter: Franke, Dirk
(86) Internationale Anmeldenummer: PCT/DE2018/100548
(87) Internationale Veröffentlichungsnummer: WO 2018/224099

(56) Entgegenhaltungen:
- EP-A2- 2 018 835
- EP-A2- 2 018 835
- WO-A1-99/36032
- WO-A1-99/36032
- DE-A1- 10 114 382
- DE-A1- 10 114 382
- DE-T2- 69 516 553
- DE-T2- 69 516 553
- KR-B1- 100 871 282
- US-A- 5 968 533
- US-A- 5 968 533
- US-A1- 2005 208 116
- US-A1- 2005 208 116

## Beschreibung

Die vorliegende Erfindung betrifft ein Silikonpad, umfassend eine Haftschicht, die im Wesentlichen aus Silikon besteht und den Wirkstoff enthält, und weiterhin umfassend einen Silikonkörper.

Die vorliegende Erfindung betrifft zudem ein kosmetisches Verfahren, umfassend das Aufbringen eines Silikonpads auf die Haut.

### Stand der Technik

Aktuelle Silikonpads basieren auf der Erkenntnis, dass Falten durch eine, beispielsweise über Nacht, angewendete, längere Tragedauer mechanisch geglättet werden können. Die Hautstruktur verbleibt über mehrere Stunden in der geglätteten Position und kann sich so regenerieren. Derartige Pads oder Klebepflaster zum Kleben auf die menschliche Haut, um damit einer Faltenbildung zu begegnen, sind allgemein bekannt. Zumeist werden diese am Dekolleté angewendet.

Die deutsche Gebrauchsmusterschrift DE 20 2014 001 876 U1 offenbart ein Dekolleté- Silikonpad zur Faltenreduzierung, das zu 100% aus medizinischem Silikon (mit den Maßen ca. 20 x 20 x 2,5 cm) besteht und über eine selbsthaftende Auflagefläche verfügt. Die selbsthaftende Auflagefläche erhält dabei durch spezielle Bearbeitungsschritte (Härtegrad des Silikons und polierte Seite als Auflagefläche) eine selbsthaftende Eigenschaft. Das Dekolleté-Silikonpad ist somit für mehrere Monate nutzbar und verliert seine Eigenschaften nicht.

Die CA 2,575,534 offenbart eine Einheit zur Faltenreduzierung, anzuwenden zwischen den weiblichen Brüsten, am Dekolleté sowie im oberen Brustbereich, die eine halb-steife/halb-flexible Membran, eine weiche Schicht sowie eine weitere Haftschicht umfasst.

Die deutsche Gebrauchsmusterschrift DE 202015106000 U1 offenbart ein Dekolletépad zum Kleben auf der Haut mit einer Stützschicht und einer Klebeschicht. Das Dekolletépad ist dabei vorzugweise spiegelsymmetrische entlang einer Symmetrieachse und tropfenförmig geformt.

Nachteilig an den vorangehend genannten Hilfsmitteln ist, dass diese nur eine rein mechanische Glättung der Haut bewirken, sodass dessen Wirkung sofort nachlassen kann, wenn die Anwendung beendet und das Hilfsmittel von der Haut entfernt wird. Zusätzlich dienen solche Hilfsmittel ausschließlich zur Faltenreduzierung bzw. als Anti-Aging-Produkt, nicht aber als Hautpflegemittel oder zur Reduzierung von Alters- und Pigmentflecken, Cellulite, Akne, Neuodermitis oder anderen Hautstörungen. Weiterhin nachteilhaft ist, dass diese Hilfsmittel nur auf einer speziellen Hautpartie anwendbar sind, insbesondere am Dekolleté und Brustbereich, nicht aber auf weiteren Hautpartien, für die aber ebenso eine Pflegewirkung sowie Bekämpfung und Vorbeugung der Hautalterung gewünscht wird.

Die Übersetzung einer europäischen Patentschrift DE 695 16 553 T2 offenbart überdies einen heiß schmelzenden Silikon-Haftkleber mit siloxylierten Polyether-Wachsen als Additiv. Die hierin offenbarten Schmelzsilicon-PSA-Zusammensetzungen weisen hydrophile Eigenschaften unter Aufrechterhaltung der PSA-Eigenschaften bezüglich Scherung, Haftung und Ablösbarkeit auf.

Die deutsche Offenlegungsschrift DE 101 14 382 A1 betrifft eine feuchtigkeitsaufnehmende Matrix auf Silikonbasis insbesondere zur Wundversorgung und/oder pharmazeutischen Hautbehandlung wobei die haftklebrige Matrix aus Silikon, Gelbildner und gegebenenfalls einem Silikonharz besteht.

Die US 2005/0208116 A1 offenbart transdermale Abgabevorrichtungen für die transdermale Abgabe einer aktiven Substanz, zum Beispiel einer pharmakologisch aktiven Substanz. Die Vorrichtung weist eine erste und eine zweite übereinander angeordnete miteinander in Kontakt stehende Haftschichten auf, von denen die erste Schicht ein druckempfindlicher Klebstoff ist und im Gebrauch in Kontakt mit der Haut gebracht wird. Die erste Schicht und die zweite Schicht können z.B. aus Silikonelastomeren bestehen.

Die koreanische Patentschrift KR 100 871 282 B1 offenbart ein kosmetisches Produkt, ein sogenanntes "Beauty Pad", das zur Verbesserung von Falten im Gesicht eingesetzt wird. Es handelt sich dabei um ein Pad, das eine Haftschicht auf einer Seite hat, sodass es auf die Gesichtshaut aufgebracht werden kann. Die andere Seite besteht aus einem Silikonblatt, das mit einem Wirkstoff zur Faltenverbesserung beschichtet ist.

### Darstellung der Erfindung, Aufgabe, Lösung, Vorteile

Ausgehend von den vorangehend genannten Überlegungen des Standes der Technik liegt der vorliegenden Erfindung deshalb die Aufgabe zugrunde, ein Silikonpad bereitzustellen, welches die oben beschriebenen Nachteile des Standes der Technik überwindet. Die der Erfindung zugrunde liegenden Aufgabe wird durch die in den Ansprüchen definierten Silikonpads gelöst, wie sich auch aus den beiliegenden Ausführungsbeispielen ergibt. Insbesondere liegt der vorliegenden Erfindung die Aufgabe zugrunde, ein Silikonpad bereitzustellen, welches es erlaubt, die Haut in einer entspannten und geglätteten Position über einen längeren Zeitraum mit dem in das Silikonpad eingearbeiteten Wirkstoff zu versorgen.

Überraschenderweise haben die Erfinder der vorliegenden Erfindung herausgefunden, dass der Wirkstoff vom Silikonpad zur Haut diffundieren, von dieser aufgenommen und dort eine biologische Wirksamkeit entfalten kann. Diese besonders vorteilhafte Form der Hautbehandlung findet sowohl in der Anti-Aging- oder Pflegebehandlung als auch bei der Behandlung von Alters- und Pigmentflecken, Cellulite, Pigmentflecken und anderen Hautstörungen wie der Wundheilung bzw. Wundheilungsstörungen oder Neurodermitis eine Anwendung. Zudem können auch andere Wirkstoffe, mitunter pharmazeutische Wirkstoffe oder Nikotin zur Rauchentwöhnung eingearbeitet werden. Die Haut wird so nicht nur durch das Gewicht des Silikonpads mechanisch geglättet, sondern gibt zudem Wirkstoffe ab, die besonders vorteilhaft zur Hautregeneration bzw. der Behandlung der Erkrankung beitragen.

Überraschenderweise können die Wirkstoffe auch nach dem Beenden der Anwendung weiter in der Haut wirken. Weiterhin kann durch Kombination des Silikonpads mit dem Wirkstoff die Kontaktfähigkeit und Aufnahmefähigkeit der mit dem Silikonpad gestrafften Haut erhöht und so eine ideale Resorption des Wirkstoffes in die Haut erreicht werden. Zwischen dem Silikonpad und der Hautschicht entsteht ein Mikroklima, welches der Haut unter anderem hilft, die Poren zu öffnen, den Stoffwechselprozess anzuregen und Wirkstoffe besser aufzunehmen.

Die über Stunden optimal geglättete Haut befindet sich durch das Silikonpad zudem in einer optimalen Versorgungslage, da die Lederhautschicht entlastet und die Blutversorgung deutlich verbessert wird. So können wieder leistungsfähige, gesunde Hautzellen gebildet werden, welche auch über eine gute Aufnahmefähigkeit für die Wirkstoffe verfügen. Die Haut wird in dieser optimal geglätteten Position langfristig mit den wichtigen Wirkstoffen versorgt und kann so eine bessere Regeneration und Faltenglättung erzielen. Auch hinsichtlich Alters- und Pigmentflecken wirkt sich auch hier die langfristige Anwendung der Wirkstoffe mittels der Verarbeitung in einem Silikonpad auf die Haut äußert positiv aus, da die Wirkstoffe länger und intensiver eindringen und somit eine optimale Wirkung erzielen können.

Das Silikonpad gemäß der vorliegenden Offenbarung kann auch in der Narbenbehandlung angewendet werden. Hierbei kann das mit mindestens einem Wirkstoff versehene Silikonpad auf Narben aufgebracht werden und die Haut durch ein Mikroklima zur Selbstheilung und schnelleren Zellerneuerung angeregt werden.

Die vorliegende Erfindung kann durch ihre unterschiedliche Formgebung in verschiedenen Ausführungsformen auf sämtlichen Hautpartien angewendet werden, insbesondere auch auf dem Handrücken, dem Hals, dem Dekolleté, der Stirn, unter den Augen und um die Mundpartie herum. Außerdem an den Oberarmen, dem unteren Kinn sowie der Region im Schambereich, wo üblicherweise Kaiserschnitte erfolgen.

In einem ersten Aspekt betrifft die vorliegende Offenbarung ein Silikonpad (1) geeignet zum Kleben auf der Haut, wie in Anspruch 1 definiert.

Im Sinne der Offenbarung ist ein Silikonpad ein aus überwiegend Medical Grade Silicone (medizinisches Silikon) bestehendes, flaches Objekt, das in zwei Raumrichtungen deutlich größere Abmessungen als in einer dritten Raumrichtung aufweist. Die beiden Raumrichtungen mit deutlich größeren Abmessungen können dabei verschiedene Formen ergeben, insbesondere einen Kreis bzw. unterschiedliche Kreisformen auch mit Kurven, eine Ellipse, ein Vieleck, eine Tropfen- oder Hand-Form.

Vorzugsweise ist das Silikonpad ein additionsvernetztes 2-komponentiges Silikon, welches sich zur Herstellung eines Silikonpads mit guter Abgabe des Wirkstoffes besonders gut eignet. Dieses Material enthält vinylterminierte Polydimethylsiloxane (CAS-No: 68083-19-2) sowie weitere Hilfsstoffe zur Vernetzung. Der Siliconanteil ist > 95%. Vorzugsweise handelt es sich bei den Siliconen um raumtemperaturvernetzende, platinkatalysierte Siliconelastomere.

Vorzugsweise ist das Silikon durch eine hohe Gas- und Wasserdurchlässigkeit gekennzeichnet. Hierdurch kann der Wirkstoff optimal durch das Silikonpad transportiert werden. In einer alternativen Implementierung ist das Silikonpad im Wesentlichen gasdurchlässig, insbesondere wasserdampfdurchlässig, jedoch durch Wasser in flüssiger Form nicht leicht zu durchdringen (wasserdicht).

Vorzugsweise ist das Silikon ein additionsvernetzendes Silikon, das zu einem weichen, klebrigen Silikonkleber ausgehärtet ist. Weitere Vorteilhafte Eigenschaften dieses Silikons sind sehr gute Biokompatibilität, keine Nebenprodukte, gute Haftung und gute Platinvernetzbarkeit.

Ein Wirkstoff ist eine körpereigene oder körperfremde Substanz, die eine Veränderung in einem biologischen Vorgang, wie beispielweise der Hautalterung, hervorrufen und diesen beeinflussen kann. Ein Wirkstoff kann auch ein pharmazeutischer Wirkstoff sein. In der Regel ist dessen Aufnahme über die Haut vorteilhaft für den Anwender.

Aufgrund des Wirkstoffes, der während der Behandlung mit dem Silikonpad in die Haut eindringen kann, kann die Haut nicht nur mechanisch durch das Gewicht und die Adhäsion des Silikonpads (1) geglättet und gepflegt werden, sondern kann zusätzlich durch den Wirkstoff behandelt werden, wobei durch die Glättung im Hinblick auf die Pharmakokinetik symbiotische Effekte beobachtet werden können.

Besonders vorteilhaft ist, dass die Haut auch nach dem Entfernen des Silikonpads (1) von der Haut durch den bereits eingedrungenen Wirkstoff weiter behandelt und dadurch eine zusätzliche Pflege der Haut gewährleistet wird. Dadurch kann eine verbesserte Hautpflege und Anti-Age-Wirkung erreicht werden. Die Kombination des Silikonpads mit dem Wirkstoff ermöglicht außerdem eine maximale Kontaktoberfläche zwischen Haut und Silikonpad und eine besonders vorteilhafte Resorption des Wirkstoffes in die Haut.

Die Bestandteile der Wirkstoffe können durch die Haut in das Gewebe eindringen und dort ihre biologische Wirksamkeit entfalten. Die Silikonpads gemäß der vorliegenden Offenbarung hat keine negativen Effekte, insbesondere stört das Tragen des Silikonpads nicht, es treten keine Peel-Effekte auf. Das Silikonpad fühlt sich weich an, die Falten werden sichtbar reduziert und die Hautverträglichkeit ist besonders gut.

Ohne an eine Theorie gebunden sein zu wollen, glauben die Erfinder der vorliegenden Erfindung, dass durch die hohe Polarität von Hyaluron und anderen Wirkstoffen und durch die geringe Polarität von Silikon das im Haftgel eingebettete Hyaluron bzw. anderen Wirkstoffe langsam an die Außenhaut, welche auf der Haut aufliegt, abgegeben wird, so dass das bereits an die Haut abgegebene Hyaluron wieder aufgefüllt werden kann. Zudem werden andere Teile des Wirkstoffes auch in Richtung Basismaterial wandern, weil keine Sperrschicht vorhanden ist. In Vergleichsstudien konnte gezeigt werden, dass Öl auch in Wasser zerstäubt, sich nach einiger Zeit als Öltropfen wieder zusammenschließt. Ohne an eine Theorie gebunden sein zu wollen, ist nach Ansicht der Erfinder ein wesentlicher Effekt der Partikelauslösung auf die Wärme und Feuchtigkeit der Haut zurückzuführen, welche unter dem Silikonpad als Mikroklima entsteht.

Vorzugsweise hat das Silikonpad eine Dicke von 0,5 bis 2,0 mm, besonders bevorzugt von 0,8 bis 1,5 mm, insbesondere im Wesentlichen 1,2 mm. Bei einer solchen Dicke des Silikonpads ist der Tragekomfort und die vermittelte Einwirkung des Wirkstoffes ideal.

Die Konzentration des Wirkstoffes im Silikonpad liegt vorzugsweise bei ca. 0,5 bis 3,0 ‰ (w/w), besonders bevorzugt bei 1,0 bis 2,0 ‰ (w/w), idealerweise bei ca. 1,6 ‰ (w/w).

In einer alternativen Implementierung kann der Wirkstoff auch in einer Konzentration von 0,1 bis 9,9 %; vorzugsweise von 0,5 bis 7,0% und idealerweise ca. 1,0 % (w/w) in dem Silikonpad enthalten sein. In diesem Fall können auch Öle, wie beispielsweise Kokosöl, Olivenöl oder Aloe Vera in flüssiger Form als Wirkstoff verwendet werden. Das Silikonpad wird überraschenderweise in diesem Fall weicher, was den zusätzlichen Vorteil einer guten Haftung auf der Haut bereitstellt.

Vorteilhafterweise kann das Silikonpad auch zusätzlich ein Silikonöl enthalten. Sofern das Silikonpad ein Silikonöl enthält, wird das Pad weicher in der Anwendung, so dass eine gute Haftung auf der Haut ermöglicht wird. Als Silikonöle im Sinne dieser Offenbarung können vorzugsweise polymerisierte Siloxane mit organischen Seitenketten verwendet werden. Diese zeichnet sich durch die periodisch alternierende Anordnung von Silicium- und Sauerstoffatomen mit der allgemeinen Summenformel [R¹R²SiO]ₙ aus. An den freien Außenelektronen des Siliciums hängen vorzugsweise Reste **R,** welche zumeist organische Reste sind, aber auch Halogene sein können. Die Molekülmasse der verwendeten Silikonöle liegt vorzugsweise im Bereich 162 bis 150.000 g/mol. Die Dichte der Silikonöle liegt vorzugsweise im Bereich von 0,76 bis 1,07 g/cm³.

In einer vorteilhaften Implementierung haben die Silikonöle eine Viskosität von 0,6 bis 1 000 000 mPs. Hierdurch wird eine optimale haptische Eigenschaft des Silikonpads erreicht.

Vorzugsweise ist das Silikonöl Polydimethylsiloxan, in denen die Reste R¹ und R² je eine Methylgruppe ist.

In einer bevorzugten Implementierung kann das Silikonpad (1) eine Haftschicht (2) und einen Silikonkörper (3) umfassen. Die Haftschicht (2) kann im Wesentlichen aus Silikon bestehen und den Wirkstoff enthalten.

Alternativ kann das Silikonpad (1) aus einem Silikonkörper (3) bestehen, welcher den Wirkstoff umfasst. Das Silikonpad kann eine polierte Seite aufweisen. In diesem Fall weist das Silikonpad eine langfristigere Selbsthaftung auf. Das Silikonpad bzw. die Haftschicht kann weitere Hilfsmittel umfassen, die eine sichere Befestigung des Silikonpads auf der Haut ermöglichen. Beispielsweise kann das Silikonpad an einer Seite einen Baumwollstoff umfassen, der das Silikonpad vor äußeren Einflüssen schützt. Zudem kann das Hilfsmittel auch ein Baumwollhandschuh sein, welcher zum Überstreifen für einen Handrücken geeignet ist.

Eine Haftschicht ist jede Materialschicht, die nach Anbringen des Silikonpads auf der Haut dessen Haftung auf der Haut auch ohne weitere Kraftzufuhr für mindestens drei Stunden oder mehr gewährleistet. Insbesondere kann eine Haftschicht im Wesentlichen aus Silikon bestehen. Vorzugsweise weist die Haftschicht einen Silikonanteil von mindestens 95% auf.

Vorzugsweise hat der Silikonkörper eine Dicke von 0,5 bis 2,0 mm, besonders bevorzugt von 0,8 bis 1,5 mm, insbesondere im Wesentlichen 1,2 mm. Die Haftschicht hat vorzugsweise eine Dicke von 0,2 bis 1,0 mm, besonders bevorzugt von 0,4 bis 0,8 mm, insbesondere 0,6 bis 0,7 mm. Bei einer solchen Dicke des Silikonkörpers und der Haftschicht ist die Dosierungsmenge des Wirkstoffes ökonomisch vertretbar und der Tragekomfort des Silikonpads ideal.

Vorzugsweise ist die Haftschicht des Silikonpads weicher als der Silikonkörper. Zudem hat vorzugsweise die Haftschicht eine höhere Wasserdurchlässigkeit als der Silikonkörper. In diesem Fall kann der Silikonkörper des Silikonpads als mechanische Stützmatrix verwendet werden während gleichzeitig die Haftschicht den Wirkstoff gut an die Haut abgeben kann.

Die Konzentration des Wirkstoffes in der Haftschicht liegt vorzugsweise bei ca. 0,6 bis 5,0 ‰ (w/w), besonders bevorzugt bei 1,5 bis 3,0 ‰ (w/w), idealerweise bei ca. 1,7 ‰ (w/w). Bei einer solchen Konzentration des Wirkstoffes in der Haftschicht ist die Diffusionsgeschwindigkeit im Verhältnis zum Materialverbrauch des Wirkstoffes ideal.

Sofern die Haftschicht (2) des Silikonpads (1) den Wirkstoff enthält, kann eine maximale Wirkstoffabgabe an die darunterliegende Haut sichergestellt werden. Die Haftschicht kann vorzugsweise bei der Anwendung in direktem Kontakt mit der Hautoberfläche stehen und so noch schneller, intensiver und länger von der Haut aufgenommen werden kann. Dies ermöglicht eine besonders effektive Behandlung der Haut. Zudem bleiben dadurch keine Wirkstoffe ungenutzt, indem diese im Silikonpad (1) verbleiben. Das Silikonpad in dieser Ausgestaltung ist insofern besonders vorteilhaft, dass durch den Silikonkörper (3) die benötigte Stabilität und das notwendige Gewicht gegeben sind, um die Haut mechanisch zu glätten. Gleichzeitig ist die Dosierung des Wirkstoffs besonders ökonomisch.

In einer weiteren Implementierung kann das Silikonpad (1) der Form und Größe nach für die Auflage auf einem Handrücken (4) geeignet sein.

Das zur Auflage auf dem Handrücken geeignete Silikonpad (1) kann dabei vorzugsweise eine Finger-Form aufweisen, wobei sich das Silikonpad (1) über den gesamten Handrücken (4) sowie über die Fingergrundgelenke (5) hinaus bis zu den Fingermittelgelenkten (6) erstrecken kann. Hierdurch kann die Hautpartie des Handrückens (4) optimal behandelt werden. Des Weiteren kann das Silikonpad (1) in den Bereichen der Fingergrundgelenke (5) flacher sein als im Bereich des restlichen Handrückens (4). Dies ermöglicht mehr Anpassungsfähigkeit, Flexibilität und Dehnbarkeit des Silikonpads (1) in diesen Bereichen, sodass sich das Silikonpad (1) trotz Bewegung der Hand von dieser nicht löst, sondern einen optimalen Halt gewährleistet. Der Vorteil dabei ist, dass das Silikonpad (1), verglichen mit bereits bekannten Produkten, nicht nur auf dem Dekolleté, dem Hals oder auf dem Gesicht, sondern auch auf anderen Hautpartien, wie dem Handrücken, angewendet werden kann. Die Anwendung eines Silikonpads mit Wirkstoffen auf dem Hals, Dekolleté oder Gesicht realisiert jedoch insbesondere bei der Hautpflege und zur Anregung der Regeneration und Faltenreduzierung auch erhebliche Vorteile zu den bisher nur manuell straffenden Silikonpads.

Das Silikonpad bzw. die Haftschicht des Silikonpads kann auch weitere Inhaltsstoffe umfassen. Beispielsweise kann das Silikonpad bzw. die Haftschicht des Silikonpads eines oder mehrere der folgenden weiteren Inhaltshoffe umfassen: Neroli; Neroliöl ; Rosmarin; Rhealba; Kupfersulfat, Kupfer, Zinksulfat, Zink; Glyzerin; Beinwell; Hamamelis oder Peptide.

Vorteilhaft daran ist, dass der Wirkstoff nach Aufkleben des Silikonpads (1) auf die Haut in direktem Kontakt mit der Hautoberfläche ist und so noch schneller, intensiver und länger von der Haut aufgenommen werden kann.

Der Wirkstoff kann dabei als feines Pulver oder als flüssige oder gelartige Masse verarbeitet werden.

In einem weiteren Aspekt umfasst die Offenbarung ein Silikonpad (1) der Form und Größe nach geeignet für die Auflage auf einen Handrücken (4), wobei das Silikonpad zur Straffung von Falten auf dem Handrücken (4) geeignet ist.

Das zur Auflage auf dem Handrücken (4) geeignete Silikonpad (1) kann dabei eine Finger-Form aufweisen, wobei sich das Silikonpad (1) über den gesamten Handrücken (4) sowie über die Fingergrundgelenke (5) hinaus bis zu den Fingermittelgelenkten (6) erstrecken kann. Dadurch kann die Hautpartie des Handrückens (4) optimal behandelt werden.

In einer weiteren Implementierung kann das Silikonpad (1) Gelenkbereiche (7) umfassen. Ein Gelenkbereich ist ein Teilbereich des Silikonpads welcher eine geringere Materialstärke aufweist und so im Verhältnis zu anderen Teilbereichen des Silikonpads leichter umknickbar ist.

Dies ermöglicht eine verbesserte Anpassungsfähigkeit, Flexibilitätund Dehnbarkeit des Silikonpads (1) in diesen Bereichen, sodass sich das Silikonpad (1) trotz Bewegung der Hand von dieser nicht löst, sondern einen optimalen Halt gewährleistet.

In einem weiteren Aspekt betrifft die Offenbarung zudem ein kosmetisches Verfahren, wie in Anspruch 9 definiert. Durch das kosmetische Verfahren wird die Haut zum einen mechanisch behandelt und gestrafft, zum anderen kann der Wirkstoff des Silikonpads in das Gewebe der Haut eindringen und dort kosmetisch wirken.

Dadurch kann eine Vorbeugung und Reduzierung von Falten sowie Alters- und Pigmentflecken erreicht werden. Außerdem kann das Silikonpad zur Hautpflege, Wund- und Narbenbehandlung, bei Hautkrankheiten, Cellulite oder generell zur Abgabe pharmazeutischer Wirkstoffe dienen. Durch unterschiedliche Ausführungsformen kann das Silikonpad an verschiedenen Hautpartien angewendet werden, insbesondere auch auf dem Handrücken.

Zudem betrifft die Offenbarung auch ein kosmetisches Verfahren zur Narbenrückbildung bei einem Probanden, wie in Anspruch 9 definiert.

Das Silikonpad kann als Wirkstoff auch weitere medizinische Wirkstoffe enthalten. Als beispielhafte medizinische Wirkstoffe seien Antibiotika,

Protonenpumpenhemmer, Antidepressiva, Schmerzmittel, Antihistaminika, Beruhigungsmittel, Wirkstoffe gegen Bluthochdruck und sonstige medizinische Wirkstoffe genannt.

### Kurze Beschreibung der Figuren

Im Folgenden werden beispielhaft und nicht abschließend einige besondere Ausführungsformen der Erfindung unter Bezugnahme auf die beiliegenden Figuren beschrieben.

Die besonderen Ausführungsformen dienen nur zur Erläuterung des allgemeinen erfinderischen Gedankens, jedoch beschränken sie die Erfindung nicht.

Es zeigt die:
Fig. 1 einen seitlichen Querschnitt eines Silikonpads (1).
Fig. 2 eine Draufsicht auf ein zur Auflage auf dem Handrücken geeignetes Silikonpad (1) in Finger-Form.
Fig. 3 zeigt das Ergebnis von Untersuchungen zur Wirkstoffaufnahme.

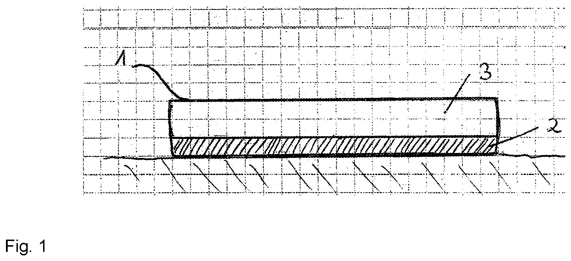

Fig. 1 zeigt einen seitlichen Querschnitt eines Silikonpads (1). Das Silikonpad (1) besteht dabei aus einem Silikonkörper (3) und einer mit Wirkstoffen versehenden Haftschicht (2), die auf der Haut aufliegt.

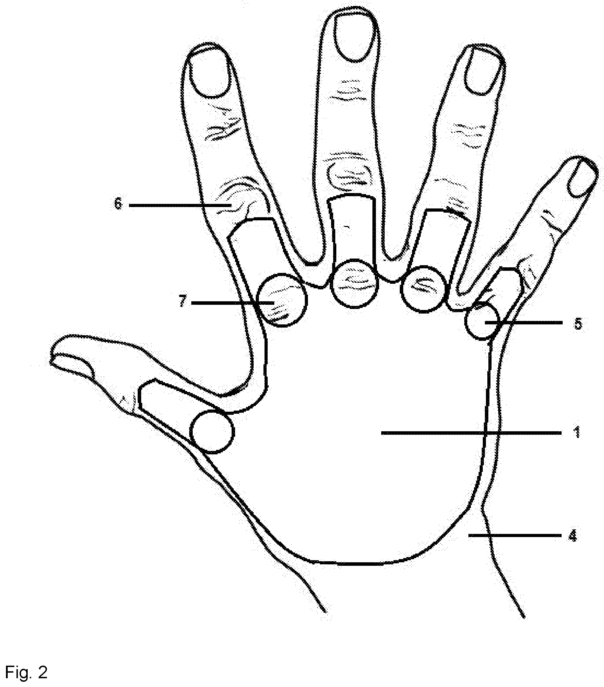

Fig. 2 zeigt eine Draufsicht eines zur Auflage auf einen Handrücken (4) geeigneten Silikonpad (1) in Finger-Form. Das Silikonpad (1) bedeckt dabei den Handrücken (4) und erstreckt sich über die Fingergrundgelenke (5) hin zu den Fingermittelgelenken (6) der Finger. Die Gelenkbereiche (7) liegen direkt über den Fingergrundgelenken (5).

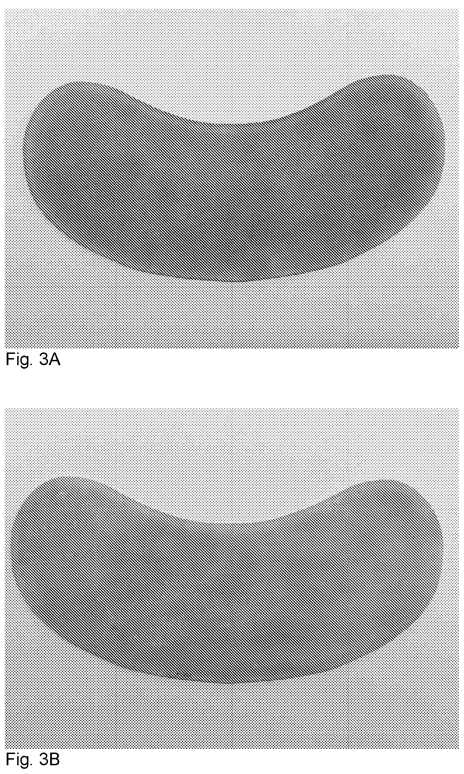

Fig. 3 zeigt das Ergebnis von Untersuchungen zur Wirkstoffaufnahme. Die Fig. 3A zeigt ein Silikonpad mit einer Haftschicht. Die Haftschicht umfasst 6 mg Hyaluron (<20T Dalton) in Pulverform. Das Pulver wurde zur Verdeutlichung der Diffusion mit einer grünen Lebensmittelfarbe eingefärbt. Die Haftschicht wurde mit einem Silikonkörper verbunden unter Herstellung eines Silikonpads. Das Silikonpad wurde über einen Zeitraum von 2 Wochen täglich auf die Haut eines Probanden aufgetragen.

Man sieht in dieser Untersuchung, dass das Hyaluron sich nicht in der oberen Schicht einlagert, sondern nach unten an die Haut abgegeben worden sein muss.

Zudem sieht man in der Untersuchung, dass die obere Silikonschicht die Trägerschicht ist. Das heißt, sie hat eine andere Festigkeit und ist im Wesentlichen völlig wasserundurchlässig. Die untere Schicht mit dem Wirkstoff ist dagegen weicher und hat eine ausreichende Wasserdurchlässigkeit, um die Partikel durch den Hautkontakt (Wärme und Feuchtigkeit) an die Haut abzugeben.

Die Fig. 3B zeigt das gleiche Silikonpad nach 2 Wochen täglicher Anwendung. Wie in der Fig. 3B deutlich zu erkennen ist, nimmt die Konzentration von Hyaluron im Silikonpad aufgrund der Diffusion in die Haut deutlich ab, was durch die deutlichen Farbrückgänge des Silikonpads belegt wird.

### Beispiele

### Herstellung von Silikonpads

Im Nachfolgenden wird beispielhaft die Herstellung von Silikonpads beschrieben, die als Dekolleté-Pad bzw. als Augen-Pad verwendet werden können.

### Information zum Herstellungsprozess

Das eingesetzte Material enthält vinylterminierte Polydimethylsiloxane (CAS-No: 68083-19-2) sowie weitere Hilfsstoffe zur Vernetzung. Der Siliconanteil ist > 95%.

Das 2-komponentige, additionsvernetzende Basismaterial wird mit einer computergesteuerten Mehrkomponentendosieranlage gleichmäßig in die entsprechende Form dosiert. Anschließend wird die Form in einen Ofen gefahren bei der es durch Strahlungswärme über eine Dauer von ca. 5-7 min und einer Temperatur von ca. 55-65°C ausvulkanisiert. Beim nächsten Schritt wird mit einer weiteren computergesteuerten Mehrkomponentendosieranlage das 2-komponentige, additionsvernetzende, klebrige Haftgel auf das ausgehärtete Basismaterial gleichmäßig dünn aufgetragen. Diese Form wird dann auch wieder in einen Ofen gefahren bei der das Haftgel ebenfalls mit Strahlungswärme ausvulkanisiert. Der Aushärtevorgang läuft über einen Zeitraum von ca. 8-10 min. bei einer Temperatur von ca. 70-85°C. Anschließend werden die fertigen Produkte entformt, kontrolliert und verpackt.

Zusetzbare Substanzen (Hyaluron, Aloe Vera...) ob flüssig oder pulverförmig, werden, um eine homogene Einarbeitung zu gewährleisten, mit einem Rührgerät mit einer Drehzahl von bis zu 25.000 U/min in eine Komponente des Haftgels eingerührt. Um eine Absetzung oder eine Entmischung der Substanz zu verhindern, wird diese Silikonkomponente immer wieder nach einem festen Turnus, abhängig von der eingerührten Substanz, aufgerührt, damit wir eine gleichbleibende Qualität gewährleisten können".

### Beispiel 1: Dekolleté-Pad

Es wurden Silikonpads aus vinylterminierten Polydimethylsiloxanen (CAS-Nr.: 68083-19-2) sowie üblichen Vernetzungsmitteln als Hilfsstoffe hergestellt. Der Silikonanteil gemäß Herstellerangaben beträgt mindestens 95 %. Die Herstellung dieser Silikonpads sind bereits an anderer Stelle beschrieben worden und entsprechen beispielsweise den in der DE 101 14 382 A1 offenbarten Matrizen auf Silikonbasis. Die Silikonpads weisen im Silikonkörper (Basismaterial) die folgenden Werte auf: Dicke 1,2 mm, Gewicht 19,3 g. Die Haftschichte weist die folgenden Werte auf: Dicke 0,6 mm, Gewicht 8,5 g. Der Gewichtsanteil des zugesetzten Hyalurons in der Haftschicht beträgt 14 mg / 8,5 g, somit 1,6 ‰ (w/w).

Die Haftschicht wird dadurch hergestellt, dass das pulverförmige Hyaluron (11 - 14 mg) in das gelförmige Silikon (8,5 g) eingerührt wird.

Das oben beschrieben Silikonpad ist geeignet zur Verwendung als Dekolleté-Pad.

### Beispiel 2: Augen-Pad

Es wurden Silikonpads aus vinylterminierten Polydimethylsiloxanen (CAS-Nr.: 68083-19-2) sowie üblichen Vernetzungsmitteln als Hilfsstoffe hergestellt. Der Silikonanteil gemäß Herstellerangaben beträgt mindestens 95 %. Die Herstellung dieser Silikonpads sind bereits an anderer Stelle beschrieben worden und entsprechen beispielsweise den in der DE 101 14 382 A1 offenbarten Matrizen auf Silikonbasis. Die Silikonpads weisen im Silikonkörper (Basismaterial) die folgenden Werte auf: Dicke 1,2 mm, Gewicht 1,9 g. Die Haftschichte weist die folgenden Werte auf: Dicke 0,7 mm, Gewicht 0,9 g. Der Gewichtsanteil des zugesetzten Hyalurons in der Haftschicht beträgt 1,5 mg / 0,9 g, somit 1,7 ‰ (w/w).

Die Haftschicht wird dadurch hergestellt, dass das pulverförmige Hyaluron (1,5 mg bis 2,0 mg) in das gelförmige Silikon (0,9 g) eingerührt wird.

Das oben beschrieben Silikonpad ist geeignet zur Verwendung unter den Augen.

### Beispiel 3: Wirksamkeitsstudie

In einer empirischen Wirksamkeitsstudie wurden erfindungsgemäße Silikonpads sowie eine Blindprobe an insgesamt 15 Probanden im Alter von 29 bis 61 Jahren getestet. Die in der Tabelle 1 aufgeführten Silikonpads wurden getestet.

**Tabelle 1: Getestete Silikonpads**

| Silikonpad Nr. | Wirkstoffe |
|---|---|
| 1 | - |
| 2 | Hyaluron |
| 3 | Kollagen |
| 4 | Aloe Vera |

Die Silikonpads wurden täglich an mindestens 14 aufeinanderfolgenden Tagen für mindestens 1 Stunde, idealerweise über Nacht, mit der Haut in Kontakt gebracht. Die Kontaktflächen waren der Handrücken, der Bereich unterhalb der Augen, der Dekolletébereich, der Hals und die Stirn. Hiernach wurden die Probanden nach ihrer subjektiven Meinung gefragt, a) ob die Silikonpads verträglich sind und ohne Probleme verwendet werden konnten, b) ob eine sichtbare Faltenreduzierung und/oder andere positive Effekte feststellbar sind, c) welche der oben genannten Silikonpads 1 bis 4 nach Wirksamkeit 1=beste, 2=sehr gute oder 3=gute Wirksamkeit zeigte und d) ob irgendwelche negative Effekte festzustellen waren.

Von den 15 Probanden waren 14 Probanden der subjektiven Meinung, dass alle Silikonpads gut verträglich waren. Ein Proband bezeichnete die Verträglichkeit als mehr oder weniger gut.

Von den 15 Probanden waren 13 Probanden der subjektiven Meinung, dass bei allen Silikonpads eine sichtbare Faltenreduzierung und/oder andere positive Effekte feststellbar waren. Zwei Probanden beschrieben, dass die sichtbare Faltenreduzierung und/oder andere positive Effekte nur bei den Silikonpads 2 bis 3 auftrat, während sie bei Silikonpad 1 unsicher waren.

Bezüglich der Wirksamkeit der Silikonpads 1 bis 4 sind die subjektiven Beobachtungen der Probanden in der Tabelle 2 zusammengefasst.

**Tabelle 2: Ergebnis der Wirksamkeitsstudie.**

| | Beobachtete Wirksamkeit | | |
|---|---|---|---|
| Silikonpad Nr. | 1 (beste) | 2 (sehr gut) | 3 (gut) |
| 1 | - | 5 | 10 |
| 2 | 15 | - | - |
| 3 | 4 | 9 | 2 |
| 4 | 1 | 8 | 6 |

Alle 15 Probanden gaben an, dass negative Effekte bezüglich der Anwendung der Silikonpads nicht feststellbar sind.

Die empirische Wirksamkeitsstudie belegt, dass die Silikonpads auf der Haut gut verträglich sind und keine negativen Effekte zeigen. Die Silikonpads mit Hyaluron als Wirkstoff zeigten die beste Wirksamkeit gegen die Faltenbildung. Auch die Silikopads mit Kollagen und, im geringeren Maße, mit Aloe Vera zeigten gute Wirksamkeit. Die schlechteste Wirksamkeit gegen die Faltenbildung zeigten die Silikonpads ohne Wirkstoff (Blindproben).

Ohne an eine Theorie gebunden sein zu wollen vermuten die Erfinder der vorliegenden Erfindung, dass durch die geschlossene bzw. vollständige Auflage des Silikonpads auf der Haut die Probanden leicht schwitzen und das Hyaluron bzw. andere Wirkstoffe durch die geringe Schichtdicke der Haftschicht abgelöst wird und durch den Schweiß aus dem Silikonmaterial eluiert wird. Auf diese Weise wirkt das Silikonpad wie ein Speicher längerfristig und beständiger auf der Hautoberfläche und kann über die ganze Tragedauer, als Mehrwegprodukt auch bei wiederholter bzw. zeitlich unterbrochener Behandlung, die gewünschte Wirkung erzielen.

Ohne an eine Theorie gebunden sein soll zu wollen vermuten die Erfinder der vorliegenden Erfindung, dass bezüglich der Wirksamkeit zur Narbenrückbildung die Silikonpads der vorliegenden Erfindung nach dem folgenden Wirkungsmechanismus wirken. Durch die Aufbringung des Silikonpads kann eine höhere Hauttemperatur erreicht werden, bei der der physiologische Prozess der Narbenheilung schneller voranschreitet. Alternativ kann die Aufbringung des Silikonpads eine höhere Luftfeuchtigkeit erreicht werden und durch diesen Prozess eine schnellere Rückbildung des Narbengewebes erreicht werden. Dieser Prozess kann durch die Abgabe entsprechender Wirkstoffen verstärkt werden.

### Beispiel 4: Studien zur Hautverträglichkeit und Hautstraffung

In einer empirischen Wirksamkeitsstudie wurden erfindungsgemäße Silikonpads an insgesamt 25 Probanden im Alter von 35 bis 64 Jahren getestet. Es wurden Silikonpads mit Hyaluron ausgeformt als Dekolleté-Pad getestet. Das Gesamtgewicht des Silikonpads betrug hierbei 28 Gramm bei einer Hyaluronzugabe von 11 mg.

Die Silikonpads wurden über einen Zeitraum von 14 aufeinanderfolgenden Tagen über Nacht mit der Haut in Kontakt gebracht. Die Kontaktfläche waren der Dekolletébereich. Hiernach wurden die Probanden nach ihrer subjektiven Meinung gefragt, ob die Silikonpads verträglich sind und ohne Probleme verwendet werden konnten. Die Hautstraffung wurde unter Verwendung eines Visioscan VC 98 im Vergleich der Werte vor der Anwendung und nach der Anwendung getestet.

Alle 25 Probanden gaben an, dass negative Effekte bezüglich der Anwendung der Silikonpads nicht feststellbar sind. Insbesondere wurde durch die Silikonpads kein Austrocknen der Haut, keine Rötung der Haut und kein unangenehmes Gefühl auf der Haut festgestellt.

Die empirische Wirksamkeitsstudie belegt, dass die Silikonpads mit Wirkstoffen (hier Hyaluron) auf der Haut gut verträglich sind, eine messbare und nachhaltige Hautglättung erzeugen und keine negativen Effekte zeigen.

Die Probanden gaben an, dass das Produkt sich nicht über Nacht von der Haut abschält Das Pad glättet die Haut auf dem Dekolleté und die Haut wird über Nacht angenehm glatt.

Hautunebenheiten und Furchen wurden über Nacht sichtbar entfernt.

Eine Hautglättung gemessen über Visioscan VC 98 zeigte, dass im Durchschnitt um 19 % geglättet wurde. Die Hautglättung wurde bei allen Probanden gleichermaßen beobachtet.

### Bezugszeichenliste

- 1.: Silikonpad
- 2.: Haftschicht
- 3.: Silikonkörper
- 4.: Handrücken
- 5.: Fingergrundgelenk
- 6.: Fingermittelgelenk
- 7.: Gelenkbereich

## Patentansprüche

1. Silikonpad (1) zum Kleben auf der Haut umfassend mindestens einen Wirkstoff zur kosmetischen Hautpflege oder gegen Hautalterung, wobei das Silikonpad (1) eine Haftschicht (2) und einen Silikonkörper (3) umfasst, wobei die Haftschicht (2) im Wesentlichen aus Silikon besteht und den Wirkstoff enthält, wobei der Wirkstoff aus hoch- und niedermolekulare Hyaluron ausgewählt ist.

2. Silikonpad (1) nach Anspruch 1, wobei das Silikonpad der Form und Größe nach für die Auflage auf einen Handrücken (4) geeignet ist.

3. Silikonpad (1) nach einem der Ansprüche 1 oder 2, wobei der Wirkstoff in einer Konzentration bei ca. 0,5 bis 3,0 ‰ (w/w), besonders bevorzugt bei 1,0 bis 2,0 ‰ (w/w), idealerweise bei ca. 1,6 ‰ (w/w) in dem Silikonpad vorliegt.

4. Silikonpad (1) nach einem der Ansprüche 1 oder 2, wobei der Wirkstoff in einer Konzentration von 0,1 bis 9,9 %; vorzugsweise von 0,5 bis 7,0% und idealerweise ca. 1,0 % (w/w) in dem Silikonpad vorliegt.

5. Verfahren zur Herstellung eines Silikonpads (1) gemäß einem der Ansprüche 1 bis 4, umfassend den folgenden Schritt: Auftragen eines Wirkstoffes auf eine Silikonmasse unter Herstellung eines Silikonpads (1).

6. Verfahren zur Herstellung eines Silikonpads (1) gemäß einem der Ansprüche 1 bis 4, umfassend die folgenden zwei konsekutiven Schritte: a) Vermischen eines Wirkstoffes mit einer Silikonmasse unter Herstellung einer Haftschicht (2) und b) Anbringung der Haftschicht an einen Silikonkörper (3) unter Herstellung eines Silikonpads (1).

7. Silikonpad (1) nach einem der Ansprüche 1 bis 4 der Form und Größe nach geeignet für die Auflage auf einen Handrücken (4), wobei das Silikonpad zur Straffung von Falten auf dem Handrücken (4) geeignet ist.

8. Silikonpad (1) nach Anspruch 7, wobei das Silikonpad (1) Gelenkbereiche (7) umfasst.

9. Kosmetisches Verfahren zur Glättung der Haut eines Probanden oder zur Narbenrückbildung bei einem Probanden, umfassend das Aufbringen eines Silikonpads gemäß einem der Ansprüche 1 bis 4, 7 oder 8 auf die Haut des Probanden.

## Claims

1. Silicone pad (1) for adhesion to the skin, comprising at least one active ingredient for cosmetic skin care or for counteracting skin aging, wherein the silicone pad (1) comprises an adhesive layer (2) and a silicone body (3), wherein the adhesive layer (2) consists essentially of silicone and contains the active ingredient, wherein the active ingredient is selected from high and low molecular weight hyaluronic acid.

2. Silicone pad (1) according to claim 1, wherein the silicone pad is shaped and sized to be suitable for placement on the back of a hand (4).

3. Silicone pad (1) according to one of claims 1 or 2, wherein the active ingredient is present in the silicone pad in a concentration of about 0.5 to 3.0 ‰ (w/w), particularly preferably 1.0 to 2.0 ‰ (w/w), ideally about 1.6 ‰ (w/w).

4. Silicone pad (1) according to one of claims 1 or 2, wherein the active ingredient is present in the silicone pad in a concentration of 0.1 to 9.9% (w/w); preferably 0.5 to 7.0%, and ideally about 1.0% (w/w).

5. Method for producing a silicone pad (1) according to one of claims 1 to 4, comprising the following step: applying an active ingredient to a silicone mass to produce a silicone pad (1).

6. Method for producing a silicone pad (1) according to one of claims 1 to 4, comprising the following two consecutive steps: a) mixing an active ingredient with a silicone mass to produce an adhesive layer (2), and b) attaching the adhesive layer to a silicone body (3) to produce a silicone pad (1).

7. Silicone pad (1) according to one of claims 1 to 4, shaped and sized for placement on the back of a hand (4), wherein the silicone pad is suitable for tightening wrinkles on the back of the hand (4).

8. Silicone pad (1) according to claim 7, wherein the silicone pad (1) comprises joint areas (7).

9. Cosmetic method for smoothing the skin of a subject or for scar reduction in a subject, comprising the application of a silicone pad according to one of claims 1 to 4, 7, or 8 to the subject's skin.

## Revendications

1. Coussin (1) en silicone destiné à être collé sur la peau, comprenant au moins un principe actif pour le soin cosmétique de la peau ou contre le vieillissement de la peau, dans lequel le coussin (1) en silicone comprend une couche (2) adhésive et un corps (3) en silicone, dans lequel la couche (2) adhésive est essentiellement composée de silicone et contient le principe actif, dans lequel le principe actif est choisi parmi l'acide hyaluronique de haut et de bas poids moléculaire.

2. Coussin (1) en silicone selon la revendication 1, dans lequel le coussin en silicone est de forme et de taille appropriées pour être appliqué sur le dos (4) de la main.

3. Coussin (1) en silicone selon l'une des revendications 1 ou 2, dans lequel le principe actif est présent dans le coussin en silicone dans une concentration d'environ 0,5 à 3,0 ‰ (p/p), particulièrement préférablement de 1,0 à 2,0 ‰ (p/p), idéalement d'environ 1,6 ‰ (p/p).

4. Coussin (1) en silicone selon l'une des revendications 1 ou 2, dans lequel le principe actif est présent dans le coussin en silicone dans une concentration de 0,1 à 9,9 % (p/p), préférablement de 0,5 à 7,0 %, et idéalement d'environ 1,0 % (p/p).

5. Procédé de fabrication d'un coussin (1) en silicone selon l'une des revendications 1 à 4, comprenant l'étape suivante: application d'un principe actif sur une masse de silicone afin de fabriquer un coussin (1) en silicone.

6. Procédé de fabrication d'un coussin (1) en silicone selon l'une des revendications 1 à 4, comprenant les deux étapes consécutives suivantes: a) mélange d'un principe actif avec une masse de silicone afin de former une couche (2) adhésive, et
b) fixation de la couche adhésive sur un corps en silicone (3) afin de former un coussin (1) en silicone.

7. Coussin (1) en silicone selon l'une des revendications 1 à 4, de forme et de taille appropriées pour être appliqué sur le dos (4) de la main, dans lequel le coussin est approprié pour resserrer les rides sur le dos (4) de la main.

8. Coussin (1) en silicone selon la revendication 7, dans lequel le coussin (1) en silicone comprend des zones (7) articulaires.

9. Procédé cosmétique pour lisser la peau d'un sujet ou pour atténuer des cicatrices chez un sujet, comprenant l'application sur la peau du sujet d'un coussin en silicone selon l'une des revendications 1 à 4, 7 ou 8.
